(19) Europäisches Patentamt
European Patent Office
Office européen des brevets

(11) EP 4 763 202 A1

(12) **EUROPEAN PATENT APPLICATION**
published in accordance with Art. 153(4) EPC

(43) Date of publication:
**24.06.2026 Bulletin 2026/26**

(21) Application number: **24868221.3**

(22) Date of filing: **13.09.2024**

(51) International Patent Classification (IPC):
***A61K 31/192*** (2006.01) ***A61J 1/05*** (2006.01)
***A61K 9/08*** (2006.01) ***A61K 31/216*** (2006.01)
***A61P 27/02*** (2006.01) ***A61P 27/10*** (2006.01)

(52) Cooperative Patent Classification (CPC):
**A61J 1/05; A61K 9/08; A61K 31/192; A61K 31/216; A61P 27/02; A61P 27/10**

(86) International application number:
**PCT/JP2024/032940**

(87) International publication number:
**WO 2025/063144 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.09.2023 JP 2023157450**

(71) Applicant: **Tsubota Laboratory, Inc.**
**Tokyo 160-8582 (JP)**

(72) Inventor: **NISHIMOTO, Akinori**
**Osaka-shi, Osaka 544-8666 (JP)**

(74) Representative: **Mewburn Ellis LLP**
**Aurora Building**
**Counterslip**
**Bristol BS1 6BX (GB)**

# (54) AQUEOUS COMPOSITION

(57) An aqueous composition includes (A) 4-phenylbutyric acid or an ester thereof, or a pharmacologically acceptable salt thereof, wherein a pH of the aqueous composition is 6.0 or more and 9.0 or less, and the aqueous composition is contained in a discharge container having a filter containing a hydrophilic material.

EP 4 763 202 A1

## Description

### Technical Field

This invention relates to an aqueous composition.

### Background Art

Sodium 4-phenylbutyrate is known to be metabolized in the body to phenylacetic acid, which conjugates with glutamic acid and is excreted in the urine, and has been used as a therapeutic agent for urea cycle disorders (Non-Patent Literature 1). Recently, sodium 4-phenylbutyrate has been reported to be useful for the prevention or treatment of ocular diseases such as myopia and presbyopia (for example, Patent Literature 1 and Patent Literature 2).

On the other hand, ophthalmic solutions may contain a preservative in order to prevent contamination by microorganisms such as bacteria after opening. However, preservatives are known to cause corneal epithelial damage due to cytotoxicity and contact dermatitis due to allergic reactions (for example, Non-Patent Literature 2). Therefore, containers have been reported that can prevent microbial contamination after opening without the addition of a preservative, by accommodating an ophthalmic solution in a container provided with a filter having a pore size smaller than that of microorganisms (for example, Patent Literature 3).

### Citation List

### Patent Literature

[Patent Literature 1] International Publication No. 2018/164113
[Patent Literature 2] International Publication No. 2020/129965
[Patent Literature 3] Japanese Unexamined Patent Publication No. 2004-051170

### Non-Patent Literature

[Non-Patent Literature 1] BUPHENYL (registered trademark) Tablets, 500 mg; BUPHENYL (registered trademark) Granules, 94%; package insert.
[Non-Patent Literature 2] Ophthalmology in Japan, Vol. 58, No. 10, pp. 945-950, 1987.

### Summary of Invention

### Technical Problem

There have been no reports regarding a storage state of sodium 4-phenylbutyrate when an aqueous composition containing sodium 4-phenylbutyrate is contained in a discharge container having a filter containing a hydrophilic material. An object of the present invention is to provide a novel aqueous composition in which a decrease in 4-phenylbutyric acid or an ester thereof, or a pharmacologically acceptable salt thereof, in the composition is suppressed while the aqueous composition is accommodated in a discharge container having a filter containing a hydrophilic material.

### Solution to Problem

As a result of intensive studies conducted to solve the above-described problem, the present inventors have for the first time found that, by setting a pH of an aqueous composition containing sodium 4-phenylbutyrate to 6.0 or more, the residual rate of sodium 4-phenylbutyrate surprisingly improves when the aqueous composition is immersed in a filter containing a hydrophilic material. The present invention is based on this finding and provides the following inventions.

[1] An aqueous composition comprising:

(A) 4-phenylbutyric acid or an ester thereof, or a pharmacologically acceptable salt thereof, wherein a pH of the aqueous composition is 6.0 or more and 9.0 or less, and the aqueous composition is contained in a discharge

container having a filter containing a hydrophilic material.

[2] The aqueous composition according to [1], wherein the hydrophilic material is hydrophilic polyethersulfone or hydrophilic polyvinylidene fluoride.
[3] The aqueous composition according to [1] or [2], wherein a pore size of the filter containing the hydrophilic material is 0.1 to 0.22 μm.
[4] The aqueous composition according to any one of [1] to [3], further comprising (B) a buffering agent.
[5] The aqueous composition according to any one of [1] to [4], further comprising (C) a chelating agent.

## Advantageous Effects of Invention

According to the present invention, it is possible to provide a novel aqueous composition in which a decrease in 4-phenylbutyric acid or an ester thereof, or a pharmacologically acceptable salt thereof, is suppressed while the aqueous composition is accommodated in a discharge container having a filter containing a hydrophilic material.

## Description of Embodiments

Hereinafter, embodiments for carrying out the present invention will be described in detail. However, the present invention is not limited to the following embodiments.
The aqueous composition according to the present embodiment contains (A) 4-phenylbutyric acid or an ester thereof, or a pharmacologically acceptable salt thereof (also simply referred to as "component (A)").

Component (A)

4-Phenylbutyric acid is also referred to as 4-PBA and is a known compound represented by the following formula:

[Chem. 1]

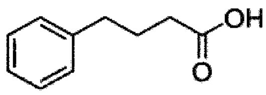

Examples of esters of 4-phenylbutyric acid include esters formed by dehydration condensation of the carboxyl group of 4-phenylbutyric acid with a monohydric alcohol having 1 to 6 carbon atoms. Specific examples include methyl esters, ethyl esters, n-propyl esters, isopropyl esters, n-butyl esters, isobutyl esters, sec-butyl esters, tert-butyl esters, n-pentyl esters, and n-hexyl esters. Among these, methyl esters, ethyl esters, n-propyl esters, and isopropyl esters are preferred.
Salts of 4-phenylbutyric acid and salts of esters of 4-phenylbutyric acid are not particularly limited as long as they are pharmacologically acceptable. Specific examples include metal salts such as sodium salts, potassium salts, calcium salts, and magnesium salts; inorganic salts such as ammonium salts; and organic amine salts such as triethylamine salts and guanidine salts. Among these, sodium salts and potassium salts are preferred, and sodium salts are more preferred.
4-Phenylbutyric acid or an ester thereof, or a pharmacologically acceptable salt thereof may be in the form of a non-solvate or a solvate (for example, a hydrate).
In the aqueous composition according to the present embodiment, an amount of component (A) is not particularly limited and is appropriately set depending on the type and the amount of other formulation components, a dosage form, and the like. From the viewpoint of more significantly exhibiting the effects of the present invention, the amount of component (A) is preferably 0.01 to 6 w/v% based on the total amount of the aqueous composition according to the present embodiment, more preferably 0.025 to 5 w/v%, still more preferably 0.05 to 4 w/v%, and particularly preferably 0.1 to 3 w/v%.

Component (B)

The aqueous composition according to the present embodiment preferably further contains (B) a buffering agent (also simply referred to as "component (B)"). By further containing the component (B), the aqueous composition more remarkably exhibits the effects of the present invention. The buffering agent includes an inorganic buffering agent and an organic buffering agent, and is not particularly limited as long as it is medically, pharmacologically, pharmaceutically, or physiologically acceptable.
An inorganic buffering agent is a buffering agent derived from an inorganic acid. Examples of inorganic buffering

agents include boric acid buffers, phosphate buffers, and carbonate buffers.

Examples of the boric acid buffer include boric acid or a salt thereof (such as an alkali metal salt of boric acid and an alkaline earth metal salt of boric acid). Examples of the phosphate buffer include phosphoric acid or a salt thereof (such as an alkali metal salt of phosphoric acid and an alkaline earth metal salt of phosphoric acid). Examples of the carbonate buffer include carbonic acid or a salt thereof (such as an alkali metal salt of carbonic acid and an alkaline earth metal salt of carbonic acid). Further, as the boric acid buffer or the phosphate buffer, a hydrate of a borate or a phosphate may also be used. More specific examples include, as the boric acid buffer, boric acid or a salt thereof (sodium borate, potassium tetraborate, potassium metaborate, ammonium borate, borax, etc.); as the phosphate buffer, phosphoric acid or a salt thereof (disodium hydrogen phosphate, sodium dihydrogen phosphate, potassium dihydrogen phosphate, trisodium phosphate, tripotassium phosphate, calcium monohydrogen phosphate, calcium dihydrogen phosphate, etc.); and as the carbonate buffer, carbonic acid or a salt thereof (sodium hydrogen carbonate, sodium carbonate, ammonium carbonate, potassium carbonate, calcium carbonate, potassium hydrogen carbonate, magnesium carbonate, etc.).

An organic buffering agent is a buffering agent derived from an organic acid or an organic base. Examples of organic buffering agents include a citric acid buffer, an acetic acid buffer, a Tris buffer, an epsilon-aminocaproic acid buffer, and an AMPD buffer.

Examples of the citric acid buffer include citric acid or a salt thereof (such as an alkali metal salt of citric acid and an alkaline earth metal salt of citric acid). Examples of the acetic acid buffer include acetic acid or a salt thereof (such as an alkali metal salt of acetic acid and an alkaline earth metal salt of acetic acid). Further, as the citric acid buffer or the acetic acid buffer, a hydrate of a citrate or an acetate may also be used. More specific examples include, as the citric acid buffer, citric acid or a salt thereof (sodium citrate, potassium citrate, calcium citrate, sodium dihydrogen citrate, disodium citrate, etc.); and as the acetic acid buffer, acetic acid or a salt thereof (ammonium acetate, potassium acetate, calcium acetate, sodium acetate, etc.). Examples of the Tris buffer include trometamol or a salt thereof (such as trometamol hydrochloride). Examples of the epsilon-aminocaproic acid buffer include epsilon-aminocaproic acid or a salt thereof. Examples of the AMPD buffer include 2-amino-2-methyl-1,3-propanediol or a salt thereof.

As the buffering agent, from the viewpoint of more prominently exhibiting the effects of the present invention, boric acid buffers (for example, a combination of boric acid and borax), phosphate buffers (for example, a combination of disodium hydrogen phosphate and sodium dihydrogen phosphate), and Tris buffers (for example, trometamol) are preferred; boric acid buffers are more preferred; boric acid and salts thereof are still more preferred; and a combination of boric acid and borax is even more preferred.

The buffering agent may be a commercially available product. The buffering agent may be used alone as one kind, or two or more kinds may be used in combination.

In the aqueous composition according to the present embodiment, an amount of component (B) is not particularly limited and is appropriately set depending on a type of the component (B), types and amounts of other formulation components, a use of the aqueous composition, a dosage form, and the like. From the viewpoint of more remarkably exhibiting the effects of the present invention, the amount of component (B) is preferably 0.05 to 5.0 w/v% based on a total amount of the aqueous composition, more preferably 0.08 to 4.5 w/v%, still more preferably 0.1 to 4.0 w/v%, and particularly preferably 0.3 to 3.5 w/v%.

In the aqueous composition according to the present embodiment, a content ratio of component (B) relative to component (A) is not particularly limited and is appropriately set depending on types of the component (A) and the component (B), types and amounts of other formulation components, a use of the aqueous composition, a dosage form, and the like. From the viewpoint of more remarkably exhibiting the effects of the present invention, relative to 1 part by mass of the total content amount of the component (A) contained in the aqueous composition according to the present embodiment, the content ratio of the component (B) relative to the component (A) is preferably 0.008 to 500 parts by mass, more preferably 0.02 to 180 parts by mass, still more preferably 0.03 to 80 parts by mass, and particularly preferably 0.1 to 35 parts by mass.

Component (C)

The aqueous composition according to the present embodiment preferably further contains (C) a chelating agent (also simply referred to as "component (C)"). By further containing the component (C), the aqueous composition more remarkably exhibits the effects of the present invention. The chelating agent is not particularly limited as long as it is medically, pharmacologically (pharmaceutically), or physiologically acceptable.

Examples of the chelating agent include ethylenediamine diacetic acid (EDDA), ethylenediamine triacetic acid, ethylenediamine tetraacetic acid (edetic acid) (EDTA), N-(2-hydroxyethyl)ethylenediamine triacetic acid (HEDTA), diethylenetriamine pentaacetic acid (DTPA), gluconic acid, and salts thereof. Examples of the salts include metal salts such as sodium salts, potassium salts, calcium salts, and magnesium salts.

As the chelating agent, edetic acid or a salt thereof is preferred, a sodium salt of edetic acid is more preferred, disodium edetate and tetrasodium edetate are still more preferred, and disodium edetate is particularly preferred.

In the aqueous composition according to the present embodiment, an amount of component (C) is not particularly limited and is appropriately set depending on a type of the component (C), types and amounts of other formulation components, a use of the aqueous composition, a dosage form, and the like. From the viewpoint of more remarkably exhibiting the effects of the present invention, the amount of component (C) is preferably 0.001 to 12 w/v% based on a total amount of the aqueous composition, more preferably 0.003 to 8 w/v%, still more preferably 0.006 to 4 w/v%, and particularly preferably 0.01 to 2 w/v%.

In the aqueous composition according to the present embodiment, a content ratio of component (C) relative to component (A) is not particularly limited and is appropriately set depending on types of the component (A) and the component (C), types and amounts of other formulation components, a use of the aqueous composition, a dosage form, and the like. Relative to 1 part by mass of the total content amount of the component (A) contained in the aqueous composition according to the present embodiment, the content ratio of the component (C) relative to the component (A) is preferably 0.0002 to 1200 parts by mass, more preferably 0.0006 to 320 parts by mass, still more preferably 0.002 to 80 parts by mass, and particularly preferably 0.003 to 20 parts by mass.

The aqueous composition according to the present embodiment may contain, in addition to the above-described components, appropriate amounts of components selected from various pharmacologically active components and physiologically active components in combination, as long as the effects of the present invention are not impaired. Such components are not particularly limited, and examples thereof include anti-allergic agents, antihistamines, anti-inflammatory agents, steroid agents, decongestants, ocular muscle regulating agents, vitamins, amino acids, astringents, and the like.

The aqueous composition according to the present embodiment may, as long as the effects of the present invention are not impaired, contain appropriate amounts of one or more various additives, which are appropriately selected in accordance with the use and dosage form thereof in accordance with conventional methods. Examples of such additives include carriers, pH adjusters, surfactants, fragrances or cooling agents, thickeners, stabilizers, preservatives, tonicity agents, and the like.

The pH of the aqueous composition according to the present embodiment is 6.0 or more and 9.0 or less. By setting the pH within this range, the effect of suppressing a decrease in the component (A) is remarkably improved. From the viewpoint of more remarkably exhibiting the effects of the present invention, the pH of the aqueous composition is preferably 8.5 or less and more preferably 8.0 or less. Further, the pH of the aqueous composition is preferably 6.5 or more and more preferably 7.0 or more.

The aqueous composition according to the present embodiment can be adjusted, as necessary, to an osmotic pressure ratio within a range acceptable to a living body. An appropriate osmotic pressure ratio may be appropriately set depending on a use, a dosage form, a method of use, and the like of the aqueous composition, and can be, for example, 0.4 to 5.0. The osmotic pressure ratio is defined, based on the Eighteenth Edition of the Japanese Pharmacopoeia, as the ratio of the osmotic pressure of a sample to 286 mOsm (the osmotic pressure of a 0.9 w/v% aqueous sodium chloride solution), and the osmotic pressure is measured with reference to the osmotic pressure measurement method (freezing point depression method) described in the Japanese Pharmacopoeia. The standard solution for osmotic pressure ratio measurement (0.9 w/v% aqueous sodium chloride solution) may be prepared by drying sodium chloride (Japanese Pharmacopoeia standard reagent) at 500 to 650°C for 40 to 50 minutes, allowing the dried sodium chloride to cool in a desiccator (silica gel), accurately weighing 0.900 g thereof, and dissolving the same in purified water to make exactly 100 mL, or by using a commercially available standard solution for osmotic pressure ratio measurement (0.9 w/v% aqueous sodium chloride solution).

The viscosity of the aqueous composition according to the present embodiment is not particularly limited as long as it is within a medically, pharmacologically (pharmaceutically), or physiologically acceptable range. The viscosity of the aqueous composition according to the present embodiment may be, for example, 1 to 10,000 mPa·s at 20°C as measured using a rotational viscometer (TV-20 type viscometer, manufactured by Toki Sangyo Co., Ltd.; rotor: 1°34'×R24).

The aqueous composition according to the present embodiment can be prepared, for example, by adding and mixing the component (A) and, as necessary, other contained components so as to obtain desired content amounts. Specifically, for example, the above-described components can be dissolved or suspended in purified water and the resulting solution or suspension can be sterilized, for example, by filtration sterilization.

When the aqueous composition according to the present embodiment is an ophthalmic composition, it can be used, for example, as an ophthalmic solution (also referred to as an eye drop solution or an eye drop agent; the ophthalmic solution also includes an ophthalmic solution that can be instilled while wearing contact lenses), an artificial tear solution, or an eye washing agent (also referred to as an eye washing solution or an eye washing agent; the eye washing agent also includes an eye washing agent that can be used for eye washing while wearing contact lenses). The term "contact lenses" includes hard contact lenses and soft contact lenses (including both ionic and non-ionic contact lenses, and including both silicone hydrogel contact lenses and non-silicone hydrogel contact lenses).

Since the aqueous composition according to the present embodiment contains sodium 4-phenylbutyrate as an active ingredient, it can be suitably used as a preventive agent, a suppressive agent, or a therapeutic agent for myopia.

Further, since the aqueous composition according to the present embodiment contains sodium 4-phenylbutyrate as an active ingredient, it can also be suitably used as a preventive agent, a suppressive agent, or a therapeutic agent for presbyopia.

The aqueous composition according to the present embodiment is preferably an ophthalmic composition, and more preferably an ophthalmic solution (including an ophthalmic solution that can be instilled while wearing contact lenses), since the effects of the present invention can be more remarkably exhibited. When the aqueous composition according to the present embodiment is an ophthalmic solution, the dosage and administration are not particularly limited as long as the dosage and administration exert effects with few side effects, and examples thereof include a method in which, for adults (15 years old or older) and children 7 years old or older, 1 to 3 drops, 1 to 2 drops, or 2 to 3 drops are instilled per dose, 1 to 4 times per day or 5 to 6 times per day.

Container with a Filter containing Hydrophilic Material

The aqueous composition according to the present embodiment is accommodated in a discharge container having a filter containing a hydrophilic material (hereinafter also referred to as a "container with a filter containing a hydrophilic material").

Examples of the hydrophilic material in the container with a filter containing a hydrophilic material include hydrophilic polyethersulfone (hydrophilic PES), hydrophilic polyvinylidene fluoride (hydrophilic PVDF), hydrophilic polytetrafluoroethylene (hydrophilic PTFE), nylon, polycarbonate, cellulose mixed esters, polyamide, and the like, among which hydrophilic polyethersulfone, hydrophilic polyvinylidene fluoride, and hydrophilic polytetrafluoroethylene are preferred, hydrophilic polyethersulfone and hydrophilic polyvinylidene fluoride are more preferred, and hydrophilic polyethersulfone is still more preferred. Further, the filter containing a hydrophilic material may be composed entirely of the hydrophilic material, or may be configured such that a part of the filter (for example, a surface of the filter) is composed of the hydrophilic material.

Hydrophilic polyethersulfone (hydrophilic PES), hydrophilic polyvinylidene fluoride (hydrophilic PVDF), and hydrophilic polytetrafluoroethylene (hydrophilic PTFE) refer to, for example, polyethersulfone (PES) subjected to a treatment for improving hydrophilicity (hydrophilization treatment), polyvinylidene fluoride (PVDF) subjected to a hydrophilization treatment, and polytetrafluoroethylene (PTFE) subjected to a hydrophilization treatment. The hydrophilization treatment can be performed, for example, by a method known to those skilled in the art.

The container with a filter containing a hydrophilic material may further have another filter. Examples of the other filter include a filter containing a hydrophilic material and a filter containing a hydrophobic material.

Examples of the filter containing a hydrophobic material include hydrophobic polyvinylidene fluoride (hydrophobic PVDF) and hydrophobic polytetrafluoroethylene (hydrophobic PTFE).

The pore size of the filter containing a hydrophilic material is not particularly limited as long as it is a size capable of removing microorganisms and the like, and may be, for example, 0.1 μm or more, 0.15 μm or more, or 0.2 μm or more, may be 0.22 μm or less, and may be 0.22 μm.

The material of the container with a hydrophilic material-containing filter is not particularly limited, and may be, for example, glass or plastic. When the material of the container with a hydrophilic material-containing filter is plastic, specific examples thereof include polyethylene terephthalate (PET), polybutylene terephthalate (PBT), polyethylene naphthalate, polyarylate, polycarbonate, polyethylene (PE; high-density polyethylene (HDPE), low-density polyethylene (LDPE)), polypropylene (PP), polystyrene (PS), acrylonitrile-butadiene-styrene (ABS), polymethylpentene (PMP), polyimide, copolymers of monomers constituting these, and mixtures of two or more thereof. Among these, polyethylene (PE) and polypropylene (PP) are preferred. Further, these plastics may contain additives such as stabilizers and reinforcing agents such as glass fibers.

In the container with a hydrophilic material-containing filter, at least a part of the portions that come into contact with the aqueous composition may be formed of the above-described material. Here, among the portions of the container with a hydrophilic material-containing filter that come into contact with the aqueous composition, examples include an inner surface of the container body (in a case where the container has a multilayer structure, the innermost layer) and an inner plug (nozzle). For example, in a case where the container has an inner plug (nozzle), only the inner plug (nozzle) may be formed of the above-described material (preferably polyethylene or polypropylene), portions other than the inner plug (nozzle), such as the container body, may be formed of the above-described material (preferably polyethylene or polypropylene), or all portions that come into contact with the aqueous composition may be formed of the above-described material (preferably polyethylene or polypropylene). From the viewpoint of more prominently exhibiting the effects of the present invention, it is preferred that all portions of the container with a hydrophilic material-containing filter that come into contact with the aqueous composition be formed of the above-described material (preferably polyethylene or polypropylene).

In the container with a hydrophilic material-containing filter, the filter may be provided inside the container body or may be provided inside the inner plug (nozzle).

As the container with a hydrophilic material-containing filter, a known filter-equipped container can be used, and examples thereof include a PF Delami container (manufactured by Rohto Nitten Co., Ltd.), an NP container (manufactured by Wakamoto Pharmaceutical Co., Ltd. or Nipro Corporation), and an ABAK bottle (manufactured by Laboratoires Thea). Further, the container with a filter may be an ophthalmic container, an eye-washing container, or the like, depending on the use of the aqueous composition.

The shape and capacity of the container with a hydrophilic material-containing filter are not particularly limited and may be appropriately set depending on the intended use. Further, the container may be a container accommodating an aqueous composition for multiple uses (a multi-dose type container) or a container accommodating an aqueous composition for a single use (a unit-dose type container); however, since the container is provided with a function of preventing bacterial intrusion by the filter, a multi-dose type container is preferred.

When the container with a hydrophilic material-containing filter is a multi-dose type container, for example, the capacity may be 1.5 to 7.5 mL, 2 to 6 mL, or 2.5 to 5.0 mL. Further, in a case where the container is a unit-dose type container, for example, the capacity may be 0.1 to 1.0 mL, 0.2 to 0.9 mL, or 0.3 to 0.8 mL.

Examples

Hereinafter, the present invention will be specifically described based on test examples; however, the present invention is not limited thereto. Unless otherwise specified, units of each component shown in the tables are w/v%.

Test Example 1: Evaluation of Residual Rate of Sodium 4-Phenylbutyrate

Each aqueous composition shown in Table 1 was prepared by a conventional method, 5 mL of each aqueous composition was placed in a 10 mL screw-cap glass bottle, two sheets of a hydrophilic polyethersulfone filter (pore size: 0.22 μm) having an area of approximately 4.3 $cm^2$ were immersed therein, and the resultant was stored at 25°C for 48 hours. For each aqueous composition, immediately after preparation and after storage at 25°C for 48 hours, the concentration of sodium 4-phenylbutyrate was determined by an HPLC method. In accordance with Formula 1, a residual rate of sodium 4-phenylbutyrate was calculated. The results are summarized in Table 1.

Formula 1: Residual rate of sodium 4-phenylbutyrate (%) = (concentration of sodium 4-phenylbutyrate in stored product/concentration of sodium 4-phenylbutyrate immediately after preparation)×100

The HPLC method was carried out under the following measurement conditions:

Mobile phase: 0.2% Formic acid solution/acetonitrile (3:2)

Column: Stainless-steel tube having an inner diameter of 4.6 mm and a length of 15 cm, packed with 5 μm octadecylsilylated silica gel for liquid chromatography

Detection method: Ultraviolet absorption photometer (measurement wavelength: 260 nm)

[Table 1]

| | Comparative Example 1 | Example 1 | Example 2 | Example 3 |
|---|---|---|---|---|
| Sodium 4-phenylbutyrate | 0.5 | 0.5 | 0.5 | 0.5 |
| Disodium edetate | 0.03 | 0.03 | 0.03 | 0.03 |
| Boric acid | 1 | 1 | 1 | 1 |
| Borax | 0.15 | 0.15 | 0.15 | 0.15 |

(continued)

| | Comparative Example 1 | Example 1 | Example 2 | Example 3 |
|---|---|---|---|---|
| Hydrochloric acid/Sodium hydroxide | Appropriate amount | Appropriate amount | Appropriate amount | Appropriate amount |
| Purified water | Remainder | Remainder | Remainder | Remainder |
| Total volume | 100 mL | 100 mL | 100 mL | 100 mL |
| pH | 5.5 | 6.0 | 7.5 | 8.5 |
| Residual rate (%) | 91.1 | 95.1 | 99.2 | 99.9 |

From Table 1, it was confirmed that when the pH of the aqueous composition is 6.0 or higher, the residual rate of sodium 4-phenylbutyrate is 95% or more, and the decrease in sodium 4-phenylbutyrate is sufficiently suppressed.

## Claims

**1.** An aqueous composition comprising:

(A) 4-phenylbutyric acid or an ester thereof, or a pharmacologically acceptable salt thereof, wherein a pH of the aqueous composition is 6.0 or more and 9.0 or less, and the aqueous composition is contained in a discharge container having a filter containing a hydrophilic material.

**2.** The aqueous composition according to claim 1, wherein the hydrophilic material is hydrophilic polyethersulfone or hydrophilic polyvinylidene fluoride.

**3.** The aqueous composition according to claim 1 or 2, wherein a pore size of the filter containing the hydrophilic material is 0.1 to 0.22 μm.

**4.** The aqueous composition according to claim 1 or 2, further comprising:
(B) a buffering agent.

**5.** The aqueous composition according to claim 1 or 2, further comprising:
(C) a chelating agent.

**INTERNATIONAL SEARCH REPORT**

International application No. **PCT/JP2024/032940**

**A. CLASSIFICATION OF SUBJECT MATTER**

***A61K 31/192***(2006.01)i; ***A61J 1/05***(2006.01)i; ***A61K 9/08***(2006.01)i; ***A61K 31/216***(2006.01)i; ***A61P 27/02***(2006.01)i; ***A61P 27/10***(2006.01)i

FI: A61K31/192; A61J1/05 313B; A61K9/08; A61K31/216; A61P27/02; A61P27/10

According to International Patent Classification (IPC) or to both national classification and IPC

**B. FIELDS SEARCHED**

Minimum documentation searched (classification system followed by classification symbols)

A61K31/192; A61J1/05; A61K9/08; A61K31/216; A61P27/02; A61P27/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2022/123837 A1 (TSUBOTA LABORATORY, INC.) 16 June 2022 (2022-06-16) claims, paragraphs [0044], [0055], [0056], examples, etc. | 1-5 |
| Y | SAMBROK, J. et al., Molecular Cloning, A LABORATORY MANUAL, SECOND EDITION, 1989, p. B.12 particularly, p. B.12, table B.7 | 1-5 |
| Y | JP 2005-160972 A (ROHTO PHARMACEUT CO., LTD.) 23 June 2005 (2005-06-23) claims, paragraph [0025], examples, fig. 1, etc. | 1-5 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 October 2024** | **22 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/032940**

| Patent document cited in search report | Publication date (day/month/year) | Patent family member(s) | Publication date (day/month/year) |
|---|---|---|---|
| WO 2022/123837 A1 | 16 June 2022 | EP 4257148 A1<br>claims, paragraphs [0038], [0049], [0050], examples<br>KR 10-2023-0135055 A<br>CN 116801864 A | |
| JP 2005-160972 A | 23 June 2005 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018164113 A
- JP 2020129965 A
- JP 2004051170 A


### Non-patent literature cited in the description


- *Ophthalmology in Japan*, 1987, vol. 58 (10), 945-950